# EUROPEAN PATENT APPLICATION

(11) **EP 4 840 606 A1**
(43) Date of publication of application: **18.11.2026**
(21) Application number: 25176848.7
(22) Date of filing: 15.05.2025
(51) Int. Cl.: B29B 9/02, B29C 45/00, C08H 99/00, C08L 99/00, C12P 19/00, C08J 3/24, C08J 3/12

(54) **A PROCESS AND A SYSTEM FOR TREATMENT OF GRAPE POMACE FOR FURTHER PROCESSING, A COMPOSITION AND A PROCESS FOR PREPARATION OF BIOPLASTIC COMPRISING SAID GRAPE POMACE AND PRODUCTS MADE FROM THE BIOPLASTIC**

(71) Applicant: ALFA TEL d.o.o., 2000 Maribor (SI); Mitrovic, Marko, 2000 Maribor (SI); Lesnik, Tomaz, 2000 Maribor (SI)
(72) Inventor: LESNIK, Tomaz, 2000 Maribor (SI); MITROVIC, Marko, 2000 Maribor (SI)
(74) Representative: Patentni Biro AF d.o.o.

(57) **Abstract**

The present invention belongs to the field of processes for using waste materials, such as winemaking waste. The invention relates to a process for treatment of grape pomace for further processing, a composition and a process for preparation of bioplastic comprising said grape pomace and products made from the bioplastic prepared by said process. The process for treatment of grape pomace for further processing comprises the following steps:
a) collection of grape pomace and pre-processing comprising mechanical separation of coarse particles larger than few milimetres, and treatement with an inert atmosphere to prevent oxidation and/or degradation of key components, such as polyphenols,
b) controlled drying comprising the following sub-steps of pre-drying at a temperature under 50°C, in controlled conditions inside a chamber, drying at increased temperature from 70 to 90°C, performed until the moisture of the material is below 8%, wherein during drying polyphenol amount is tracked and wherein at least 5 mg of GAE/g dry weight has to be present,
c) Mechanical processing comprising controlled grinding, wherein the dried pomace is grinded to particle size between 50-200 µm.

This process increases the stability of the material, removes impurities and improves the compatibility of the pomace with various materials for further industrial use.

## Description

### Field of the invention

The present invention belongs to the field of processes for using waste materials, such as winemaking waste. The invention relates to a process and a system for treatment of grape pomace for further processing, a composition and a process for preparation of bioplastic comprising said grape pomace and products made from the bioplastic prepared by said process.

### Background of the invention and the technical problem

During winemaking high quantities of residues are generated, which are often discarded. These wastes include vine pruning, grape stems, and grape pomace. Only recently, wine industry began considering these materials for reutilization, which can minimize the environmental issues related to waste and also lead to higher profitability. However, grape pomace is often used in low-cost applications, where its physicochemical properties are not optimally exploited. Existing processing methods do not allow for homogeneous quality and stability of the material, which reduces its usefulness in industrial processes. The present invention aims to provide methods for preparing grape pomace for further use and processing to allow efficient and environmentally friendly manufacturing of new products.

### Prior art

Different waste types, either from olive oil manufacturing or waste food have already been used in recycling or processing to manufacture new products or materials. Patent Patent US11919973B2 discloses a method for producing bioplastic granules, comprising the process steps of:
- mixing a dry olive pit waste (prina) with a NaOH solution in a reactor to obtain a first mixture,
- cooling the first mixture to obtain a cooled mixture,
- obtaining a solid cellulose by separating the cooled mixture by a filtering method, wherein a hemicellulose structure and a lignin structure are available in a dissolved state in the NaOH solution, the hemicellulose structure and the lignin structure are obtained from olive pits and are available in the cooled mixture,
- washing the solid cellulose with distilled water,
- mixing the solid cellulose with H₂SO₄ in the reactor to obtain a second mixture,
- cooling the second mixture,
- separating a cellulosic part and a shredded, dissolved cellulose part from each other in the second mixture by using a filtering paper after the cooling to obtain a separated liquid component, wherein the cellulosic part is a pellet part in the second mixture and the shredded, dissolved cellulose part is polymerized,
- adding glycerin or sorbitol and an antimicrobial agent (oleuropein) to the separated liquid component and placing the separated liquid component in a heater,
- subjecting the shredded, dissolved cellulose part in the separated liquid component to a polymerization reaction in the heater and obtaining a pulp structure,
- leaving the pulp structure to cooling at room temperature to obtain a cooled pulp structure,
- drying the cooled pulp structure.

These steps differ from the present invention.

Grape pomace has also been mentioned in preparation of polymers, for example in documents US2023242754, US2023242755, and US11279823, but with no specific disclosure about the steps necessary to process the grape pomace or the steps for its use.

According to the applicant's knowledge, the specific steps for pomace treatment and the bioplastic composition according to the invention have never been proposed.

### Description of the solution to the technical problem

The invention addresses the disadvantages of known solutions and provides a methods and systems for processing grape pomace and manufacturing of bioplastic using said grape pomace. In a further aspect, the invention relates to products made from so obtained bioplastic material. The technical problem is solved as defined in independent claims, where in preferred embodiments are defined in dependent claims.

In a first aspect of the present invention, a process for treatment or processing of grape pomace for further processing is disclosed, wherein the process comprises the following steps:
a) collection of grape pomace and pre-processing to ensure homogenization of grape pomace from different sources, which contributes to repeatability and reliability of the process, wherein
   - fresh grape pomace is obtained directly from the wineries within 48 hours after pressing to prevent unwanted fermentation and degradation of bioactive compounds,
   - mechanical separation of coarse particles larger than few milimetres, for example 2 mm or more, such as stalks, seeds and impurities, preferably using mechanical shakers and/or vibrators and/or filtration devices,
   - treatement with an inert atmosphere such as CO₂ or nitrogen to prevent oxidation and/or degradation of key components, such as polyphenols,
b) controlled drying comprising the following sub-steps:
   a) pre-drying at a temperature under 50°C, most commonly 45 ±2 °C, in controlled conditions inside a chamber, wherein the humidity is below 40% (relative humidity), air flow is either laminar or steady controlled flow with filtered air in the range from 0.5 to 1.5 m³/min per kg dry mass, which enables even drying without heat stress, and the moisture of the pomace is decreased from 55-70 % to aprox. 15-20 %, which prevents denaturation of natural polymers,
   b) drying at increased temperature from 70 to 90°C, performed until the moisture of the material is below 8%, which is needed for further storage and processing, preferably low-pressure drying technology is used, such as vacuum drying, drying with circulation and de-humifidation of air using condenstation dehumidifiers, drying in an atmosphere with lower oxygen content,
   c) wherein during drying polyphenol amount is tracked, preferably using spectrophotometric methods, to ensure suitable control of antioxidative and mechanical quality of the material, as polyphenols contribute to mechanical stability (function as cross-linking compounds), UV protection and natural antimicrobial activity, and wherein at least 5 mg of GAE/g dry weight has to be present, optimally between 8 and 15 mg GAE/g dry weight, depending on the type of pomace and drying,
      wherein in case of too high values, imperfect drying or contamination with unwanted compounds occurred, which leads to a new cycle of drying or pre-drying,
      and wherein in case of too low values, the degradation has occurred, which leads to discarding of the batch or mixing with other components for the preparation of lower quality products,
   d) preferably enzymatic treatment of the pomace, either before drying or after drying, to release polymers, improve homogeneity and reduce lignin or vax matter that could hamper mixing, wherein said enzymatic treatment is performed using at least one, preferably all of the enzymes in the group of:
      - cellulase for degrading cellulose in pomace,
      - pectinase for improving porosity and degradation,
      - lignin-modifying enzymes or laccase for breakdown of lignin,
      wherein the preferred concentration of the enzyme is 0,1-0,5 % (w/w) based on the weight of the treated pomace,
c) mechanical processing comprising the following sub-steps:
   - controlled grinding, wherein the dried pomace is grinded to particle size between 50-200 µm to ensure proper homogenisation with other components in next steps, and wherein smaller or larger particles are removed,
   - optionally, treating the obstained particles with physical or chemical methods such as plasma, such as low-temeprature plasma in O₂, N₂, or Ar atmosphere, or ionic treatment, such as corona, ozone or oxygen plasma, to affect surface properties of the particles,
d) preferably addition of further components for stabilisazion:
   - antioxidant and/or stabilizators, such as ascorbic acid, tocopherols, citric acid, gallic acid and/or esters thereof, catehines, quercetin, lignins, tannins, natural waxes such as bee vax or carnauba vax, for preventing oxidation and ensuring long-term stability of the material, optionally also allowing protection against microbial degradation and/or aid in corss-linking,
   - surface treatment with compounds improving compatibility with polymers such as PLA, PHA and similar, wherein said compounds are selected in the group comprising: citrate esters, lignins, natural waxes, tanins and glycerol monostearate,
e) optionally, the obtained material can be packed in nitrogen or CO₂ atmosphere to ensure stability and prevent microbial degradation,
f) optionally granulation of the material for further use, wherein granulation is performed in any manner known to the person skilled in the art.

This process increases the stability of the material, removes impurities and improves the compatibility of the pomace with various materials for further industrial use.

The second aspect of the present invention relates to a process for preparation of bioplastic composition comprising said treated grape pomace. Components are added either after drying and stabilization of pomace, before or after granulation, or during extrusion, if the material is shaped into granules directly from treated pomace. In any case, the pomace has to be treated, ground and stabilized. The mixing is performed in any known manner. The composition comprises:
- treated pomace obtained as described above in amount from 5 to 60% as base material, which ensures structural integrity and biodegradability, wherien the desired polyphenol concentration ensures mechanical rigidity and prevention of oxidation,
- optionally, other wine industry waste, if these do not affect the properties such as pH, colour or particle size,
- at least one base polymeric matrice selected in the group of biodegradable or recyclable thermoplastic polymers, to be combined with the treated pomace, wherein the base polymeric matrice is preferably selected in the group comprising: PLA, PHA, PBAT, PBS, Polypropylene, LDPE, HDPE, PP/PE, PPC, PPH, Polystyrene, processed waste plastic materials and similar polymeric plastic matrices, and combinations thereof, wherein the amount of base material is from 40 to 95%,
- preferably at least one natural plasticizer selected from the group consisting of vegetable oils, polyglycerides, natural waxes, in a concentration from 5 to 15%, preferably esterified fatty acids, which improve flexibility and impact/breakage resistance, improve molding properties,
- preferably at least one reinforcement agent selected in the group comprising microfibers from waste grape pomace, bamboo fibers, hemp fibers or natural nanocellulose crystals, in a concentration of 10 to 20%, to increase mechanical strength and improve structural stability,
- preferably at least one biodegradable agent, such as PLA and/or PHA, in concentration from 5 to 15% to ensure biodegradability, wherein possible agents for ensuring compatibility are added and seelcted in the group comprising:
   - citrate esters (e.g. triethyl citrate - TEC) to improve flexibility and adhesion to fibers,
   - TPU-g-MA (thermoplastic urethanes with maleic anhydride), which functions as a chemical bridge between the polar and non-polar phases,
   - lignins, which are natural compatibilizers that, in addition to compatibility, also contribute to UV protection and strength,
   - natural waxes (e.g. carnauba, candelilla) lower surface tension and improve processability,
- preferably at least one cross-linking agent in concentration from 2 to 5 % (w/w) for molecular stability and heat resistance, which improves industrial use of the material, wherein said cross-linking agents are selected in the group comprising natural or bio crosslinking agents, lignins and tannins from plant sources, lignins from pomace or other agro-waste, fermented flavonoids (e.g. catechins), and grape seed extract.

The process as described above ensures increased stability, improved compatibility and optimized composition, enabling uniform and efficient use of pomace in industrial processing. The obtained material is mechanically strong, biodegradable and suitable for commercial use in glassware, packaging and consumer products.

According to a third aspect of the present invention, a system for performing the above-described process is disclosed. The process as described above can be performed in a system allowing modular and step-wise treatment of the pomace, wherein the system comprises the following:
- a separator for separating coarse particles larger than few milimetres, for example 2 mm or more, such as stalks, seeds and impurities, preferably comprising mechanical shakers and/or vibrators and/or filtration devices,
- a pre-drying chamber and a drying chamber, wherein pre-drying and drying can also be performed in the same chamber,
- a chamber for enzymatic treatment of the pomace,
- a mechanical grinder for grinding the pomace
- optionally, a stabilization chamber arranged to allow addition of stabilizing compounds, antioxidants or compounds for surface-treatment of ground pomace,
- optionally, a packaging unit, and
- optionally, a granulation chamber.

Acording to a fourth aspect of the invention, products made from the bioplastic prepared by said process are provided. Said products are prepared by a method comprising the following steps:
- preparation of granules prepared as described above, wherein the granules are heated and have a viscosity of 200 to 450 Pa·s at 180-200 °C (measured at shear rate 100 s⁻¹), suitable for 3D printing and moulding,
- optionally, addition of further components such as anti-static agents, such as lecitine or plant tensides, UV stabilizers such as grape seed extract, fillers such as talc, chalk, silica for improved rigidity, and/or colouring agents,
- shaping of the product, performed by
   ∘ injection molding, wherein the granules are melted at a temperature from 180 to 220°C and the material is poured into moulds, wherein the material usually hardens in at least 10 seconds, depending on the thickness of walls, geometry of the product and mould temperature, or
   ∘ extrusion molding, wherein the granules are heated and shaped into plates or foils, with optional addition of UV stabilisers or antimicrobial agents if the further use of products requires such additives, or
   ∘ thermal forming, wherein the granules are heated and shaped into 3D products.

Granules can be used in production of various products, using 3D printing, CNC processing, laser cutting extrusion moulding, injection moulding and thermal forming.

### Detailed description of the invention

The process for preparation of grape pomace for further processing according to a possible embodiment comprises the following steps:
a) collection of grape pomace and pre-processing to ensure homogenization of grape pomace from different sources, which contributes to repeatability and reliability of the process, wherein
   - fresh grape pomace is obtained directly from the wineries within 24 to 48 hours after pressing to prevent unwanted fermentation and degradation of bioactive compounds,
   - mechanical separation of coarse particles larger than few milimetres, for example 2 mm or more, such as stalks, seeds and impurities, using mechanical shakers and/or vibrators and/or filtration devices,
   - treatement with an inert atmosphere such as CO₂ or nitrogen to prevent oxidation and/or degradation of key components, such as polyphenols,
b) controlled drying comprising the following sub-steps:
   e) pre-drying at a temperature 45 ±2 °C, in controlled conditions inside a chamber, wherein the humidity is below 40%, air flow is either laminar or steady controlled flow with filtered air in the range from 0.5 to 1.5 m³/min per kg dry mass, which enables even drying without heat stress, and the moisture of the pomace is decreased from 55-70 % to aprox. 15-20 %, which prevents denaturation of natural polymers,
   f) drying at increased temperature from 70 to 90°C, performed until the moisture of the material is below 8%, which is needed for further storage and processing, preferably nizkotlačna sušilna tehnologija is used, such as vacuum drying, drying with circulation and de-humifidation of air using condenstation dehumidifiers, drying in an atmosphere with lower oxygen content,
   g) wherein during drying polyphenol amount is tracked using spectrophotometric methods, to ensure suitable control of antioxidative and mechanical quality of the material, and wherein at least 5 mg of GAE/g dry weight has to be present, optimally between 8 and 15 mg GAE/g dry weight, depending on the type of pomace and drying,
   h) enzymatic treatment of the pomace, either before drying or after drying, to release polymers, improve homogeneity and reduce lignin or vax matter that could hamper mixing, wherein said enzymatic treatment is performed using at least one, preferably all of the enzymes in the group of:
      - cellulase for degrading cellulose in pomace,
      - pectinase for improving porosity and degradation,
      wherein the enzyme concentration was 0.3 % (w/w) based on the weight of the wet (pre-dried) treated pomace,
c) mechanical processing comprising the following sub-steps:
   - controlled grinding, wherein the dried pomace is grinded to particle size between 50-200 µm to ensure proper homogenisation with other components in next steps, and wherein smaller or larger particles are removed,
d) addition of further components for stabilisazion:
   - antioxidant and/or stabilizators, namely ascorbic acid and citric acid, in concentration of 0.2 %,
   - surface treatment with compounds improving compatibility with polymers such as PLA, PHA and similar, wherein said compounds were lignin and glycerol monostearate in concentration of 1% and 0.5 %, respectively.

To obtain 1 kilogram of dry material from grape pomace, it is necessary to take into account that fresh pomace comprises approximately 55-70% moisture. After the drying process to the target 8% moisture, approximately 30-45 kg of dry mass remains from 100 kg of fresh pomace. This means that for 1 kg of dry mass, approximately 2.2 to 3.3 kg of fresh pomace are needed, with the exact ratio varying depending on the initial moisture content. For calculating the air flow during drying, wherein the preferred flow rate is 0.5-1.5 m³/min per kilogram of dry mass, the ratio 1 kg of dry mass = 2.5-3 kg of fresh pomace is usually taken as a starting value.

The treated pomace is then mixed with other components to form biocomposites and then granulated for further use and/or processing.

### Example 1: A biocomposite comprising PLA, TPU-g-MA and grape pomace treated according to the invention

### Biocomposite formulations:

| **Example** | **Components and their amount** | | |
|---|---|---|---|
| | **PLA Bio-flex S7514** | **TPU-g-MA** | **Treated grape pomace** |
| Example 1a | 89.5 % | 0.5 % | 10 % |
| Example 1b | 94.5 % | 0.5 % | 5% |
| Example 1c | 83.5 % | 1.5 % | 15 % |

The biocomposite was prepared by mixing the granules of PLA with TPU-g-MA. The biocomposite was then granulated in the following manner:
- extruder Labtech LTE 20-44 with twin screw, diameter 20 mm, L:D = 44:1 was used to extrude the biocomposite material, wherein
   ∘ the temperatures from hopper to nozzle were 160, 165, 165, 170, 170, 170, 175, 175, 175, 180, 180 °C;
   ∘ speed was 400 min⁻¹;
   ∘ dosage 5 kg/h;
   ∘ and nozzle pressure approx. 20 bar
followed by water bath and granulation, wherein the obtained extruded filament is cut into pellets or granules of suitable size.

The prepared exemplary biocomposite was injection moulded under the following process parameters to allow testing of the biocomposite properties as described below. For injection moulding Krauss Maffei CX 50-180 was used. The granules were dried for 4 hours at 50 °C. The parameters of injection moulding were:
- Plasticizing back pressure: 195 bar,
- Plasticizing speed: 175 min⁻¹,
- Post-pressure: 700/1200/300 bar (1/10/2 s),
- Injection speed: 50 mm/s (last 2 mm: 20 mm/s),
- Cooling time: 10 s,
- Mold temperature: 45 °C,
- Cylinder temperature: 165 to 180 °C.

The prepared exemplary biocomposite exhibited suitable elasticity, rigidity, moisture resistance and biodegreadation, wherein the biocomposite exhibited the following properties:
- rheological properties:

| **Property** | **Value** | **Tested according to standard** |
|---|---|---|
| MFI (190°C, 2,16 kg) | 20,7 g/10 min | ISO 1133 |

- mechanical properties:

| **Property** | **Value** | **Tested according to standard** |
|---|---|---|
| Flexural modulus | 2,4 GPa | ISO 178 |
| Flexural strength | 54 MPa | ISO 178 |
| Strain at flexural strength | 3,7 % | ISO 178 |
| Tensile modulus | 2,2 GPa | ISO 527 |
| Tensile strength | 38 MPa | ISO 527 |
| Elongation at tensile strength | 2,5 % | ISO 527 |
| Elongation at break | 2,6 % | ISO 527 |
| Impact strength (Charpy, unnotched, 25 °C) | 10.0 kJ/m² | ISO 179 |
| Notched impact strength (Charpy) | 3.6 kJ/m² | ISO 179 |

- thermal properties:

| **Property** | **Value** | **Tested according to standard** |
|---|---|---|
| Glass transition temperature (Tg) | 38.8 °C | ISO 11357-3 |
| Melting temperature (Tm) | 168 °C | ISO 11357-3 |
| Degradation temperature (Td) | 328 °C | ISO 6964 |
| Carbon black content | 6.9 % | ISO 6964 |

Test products from the said biocomposite were prepared by a method comprising the following steps:
- preparation of granules prepared as described above, wherein the granules are heated and have a viscosity of 200 to 450 Pa·s at 180-200 °C (measured at shear rate 100 s⁻¹),
- shaping of the product, performed by
   ∘ injection molding for the preparation of test cups, wherein the granules are melted at a temperature from 180 to 220°C and the material is poured into moulds, wherein the material usually hardens in at least 10 seconds, depending on the thickness of walls, geometry of the product and mould temperature, or
   ∘ 3D printing for wine glasses, wherein the biocomposite granulate was pre-extruded into a 1.75 mm diameter filament, then printed with a conventional FDM 3D printer, which supports PLA filaments, and wherein a slightly higher nozzle temperature (200-210 °C) and a larger nozzle (0.6 mm) were used.

### Example 2: A biocomposite comprising PHA, maleated PHA, tocopherol and grape pomace treated according to the invention

The biocomposite formulation according to a second example comprises:
- PHA as base polymeric matrix - 85 %,
- grape pomace treated as described in example 1 = 10 %,
- maleated PHA 2 %, and
- tocopherol - 1,5 %.

### Example 3: A biocomposite comprising PBAT, citrate ester, natural vax and grape pomace treated according to the invention

The biocomposite formulation according to a third example comprises:
- PBAT as base polymeric matrix - 80 %,
- grape pomace treated as described in example 1 = 15 %,
- citrate esters - 2 %, and
- natural vax - 3 %.

### Example 4: A biocomposite comprising PLA, TPU-g-MA and grape pomace treated according to the invention

The biocomposite formulation according to a fourth example comprises:
- recycled PET as base polymeric matrix - 85 %,
- grape pomace treated as described in example 1 = 10 %,
- silane coating - 3 %, and
- ascorbic acid - 2 %.

### Example 5: A biocomposite comprising a mixture of starch and cellulose, natural tannins, gallic acid and grape pomace treated according to the invention

The biocomposite formulation according to a fifths example comprises:
- a mixture of starch and cellulose as base polymeric matrix - 70 %,
- grape pomace treated as described in example 1 = 20 %,
- natural tannins - 5 %, and
- gallic acid - 5 %.

## Claims

1. A process for treatment of grape pomace for further processing, wherein the process comprises the following steps:
a) collection of grape pomace and pre-processing to ensure homogenization of grape pomace, wherein
- fresh grape pomace is obtained directly from the wineries within 48 hours after pressing to prevent unwanted fermentation and degradation of bioactive compounds,
- mechanical separation of coarse particles larger than few milimetres, preferably using mechanical shakers and/or vibrators and/or filtration devices,
- treatement with an inert atmosphere to prevent oxidation and/or degradation of key components, such as polyphenols,
b) controlled drying comprising the following sub-steps:
i. pre-drying at a temperature under 50°C, in controlled conditions inside a chamber, wherein the humidity is below 40%, air flow is either laminar or steady controlled flow with filtered air in the range from 0.5 to 1.5 m³/min per kg dry mass, and the moisture of the pomace is decreased from 55-70 % to aprox. 15-20 %, which prevents denaturation of natural polymers,
ii. drying at increased temperature from 70 to 90°C, performed until the moisture of the material is below 8%,
iii. wherein during drying polyphenol amount is tracked, and wherein at least 5 mg of GAE/g dry weight has to be present,
c) mechanical processing comprising the following sub-step:
- controlled grinding, wherein the dried pomace is grinded to particle size between 50-200 µm to ensure proper homogenisation with other components in next steps, and wherein smaller or larger particles are removed.

2. The process according to claim 1, further comprising a step of:
iv. enzymatic treatment of the pomace, either before drying or after drying, to release polymers, improve homogeneity and reduce lignin or vax matter that could hamper mixing, wherein said enzymatic treatment is performed using at least one, preferably all of the enzymes in the group of:
- cellulase for degrading cellulose in pomace,
- pectinase for improving porosity and degradation,
- lignin-modifying enzymes or laccase for breakdown of lignin,
wherein the preferred concentration of the enzyme is 0,1-0,5 % (w/w) based on the weight of the treated pomace,

3. The process according to claim 1 or claim 2, wherein the process further comprises a step of
d) addition of further components for stabilisazion:
- antioxidant and/or stabilizators, such as ascorbic acid, tocopherols, citric acid, gallic acid and/or esters thereof, catehines, quercetin, lignins, tannins, natural waxes such as bee vax or carnauba vax, for preventing oxidation and ensuring long-term stability of the material, optionally also allowing protection against microbial degradation and/or aid in corss-linking,
- surface treatment with compounds improving compatibility with polymers such as PLA, PHA and similar, wherein said compounds are selected in the group comprising: citrate esters, lignins, natural waxes, tanins and glycerol monostearate.

4. The process according to any of the preceding claims, further comprising the steps of:
e) packaging the obtained material in nitrogen or CO₂ atmosphere to ensure stability and prevent microbial degradation, and/or
f) granulation of the material for further use.

5. The process according to any of the preceding claims, wherein the concentration of polyphenol in step b)iii is between 8 and 15 mg GAE/g dry weight.

6. The process according to any of the preceding claims, wherein
- in case of too high values, imperfect drying or contamination with unwanted compounds occurred, which leads to a new cycle of drying or pre-drying, or
- in case of too low values, the degradation has occurred, which leads to discarding of the batch or mixing with other components for the preparation of lower quality products.

7. The process according to any of the preceding claims, wherein for drying low-pressure drying technology is used, such as vacuum drying, drying with circulation and de-humifidation of air using condenstation dehumidifiers, drying in an atmosphere with lower oxygen content.

8. The process according to any of the preceding claims, wherein in step c) after grinding a further step of treating the obstained particles with physical or chemical methods is performed, such as plasma, for example low-temeprature plasma in O₂, N₂, or Ar atmosphere, or ionic treatment, such as corona, ozone or oxygen plasma, to affect surface properties of the particles.

9. A system for performing the process according to any of the preceding claims, wherein the system comprises the following:
- a separator for separating coarse particles larger than few milimetres, preferably comprising mechanical shakers and/or vibrators and/or filtration devices,
- a pre-drying chamber and a drying chamber, wherein pre-drying and drying can also be performed in the same chamber,
- a chamber for enzymatic treatment of the pomace,
- a mechanical grinder for grinding the pomace.

10. The system according to the preceding claim, further comprising:
- a stabilization chamber arranged to allow addition of stabilizing compounds, antioxidants or compounds for surface-treatment of ground pomace, and/or
- a packaging unit, and/or
- a granulation chamber.

11. A bioplastic composition comprising:
- treated grape pomace obtained with the method and/or the system according to any of the preceding claims, wherein the amount of the treated grape pomace is from 5 to 60% as base material,
- at least one base polymeric matrice selected in the group of biodegradable or recyclable thermoplastic polymers to be combined with the treated pomace, wherein the base polymeric matric is preferably is selected in the group comprising: PLA, PHA, PBAT, PBS, Polypropylene, LDPE, HDPE, PP/PE, PPC, PPH, Polystyrene, processed waste plastic materials and similar polymeric plastic matrices, and combinations thereof, wherein the amount of base material is from 40 to 95%.

12. The bioplastic composition according to the preceding claim, wherein the composition further comprises one or more of the following:
- at least one natural plasticizer selected from the group consisting of vegetable oils, polyglycerides, natural waxes, in a concentration from 5 to 15%, preferably esterified fatty acids, and/or
- at least one reinforcement agent selected in the group comprising microfibers from waste grape pomace, bamboo fibers, hemp fibers or natural nanocellulose crystals, in a concentration of 10 to 20%, and/or
- at least one cross-linking agent in concentration from 2 to 5 % (w/w), wherein said cross-linking agents are selected in the group comprising natural or bio crosslinking agents, lignins and tannins from plant sources, lignins from pomace or other agro-waste, fermented flavonoids, and grape seed extract.

13. The bioplastic composition according to claim 11 or claim 12, wherein it further comprises at least one biodegradable agent, such as PLA and/or PHA, in concentration from 5 to 15% to ensure biodegradability, wherein agents for ensuring compatibility with the PLA and/or PHA are added and selected in the group comprising:
- citrate esters, such as triethyl citrate - TEC, to improve flexibility and adhesion to fibers,
- a thermoplastic urethane with maleic anhydride (TPU-g-MA ), which functions as a chemical bridge between the polar and non-polar phases,
- lignins, which are natural compatibilizers that, in addition to compatibility, also contribute to UV protection and strength,
- natural waxes, such as carnauba, candelilla, to lower surface tension and improve processability.

14. A method for manufacturing products using the biocomposite composition
according to any claim from 9 to 13 and/or treated grape pomace using the method according to any claim from 1 to 8, wherein said method comprises the following steps:
- preparation of granules prepared as described above, wherein the granules are heated and have a viscosity of 200 to 450 Pa·s at 180-200 °C (measured at shear rate 100 s⁻¹), suitable for 3D printing and moulding,
- shaping of the product, performed by
∘ injection molding, wherein the granules are melted at a temperature from 180 to 220°C and the material is poured into moulds, wherein the material usually hardens in at least 10 seconds, depending on the thickness of walls, geometry of the product and mould temperature, or
∘ extrusion molding, wherein the granules are heated and shaped into plates or foils, with optional addition of UV stabilisers or antimicrobial agents if the further use of products requires such additives, or
∘ thermal forming, wherein the granules are heated and shaped into 3D products.

15. The method according to the preceding claim, wherein before shaping addition of further components such as anti-static agents, such as lecitine or plant tensides, UV stabilizers such as grape seed extract, fillers such as talc, chalk, silica for improved rigidity, and/or colouring agents, is performed.
